# EUROPÄISCHE PATENTSCHRIFT

(11) **EP 0 773 228 B1**
(45) Veröffentlichungstag und Bekanntmachung des Hinweises auf die Patenterteilung: **03.04.2002**
(21) Anmeldenummer: 96117361.4
(22) Anmeldetag: 29.10.1996
(51) Int. Cl.: C07K 16/00, C07K 14/05, C07K 14/72, C12N 15/86

(54) **Verfahren zur Herstellung von menschlichen monoklonalen Antikörpern und deren Verwendung**
Method of producing human monoclonal antibodies and their use
Procédé de préparation d'anticorps monoclonaux humains et leur utilisation

(30) Priorität: 09.11.1995 DE 19541844
(43) Veröffentlichungstag der Anmeldung: 14.05.1997
(73) Patentinhaber: GSF-Forschungszentrum für Umwelt und Gesundheit GmbH, 85764 Oberschleissheim (DE)
(72) Erfinder: Bornkamm, Georg Wilhelm, Prof., 81243 München (DE); Eick, Dirk, Priv. Doz. Dr., 82110 Germering (DE); Kempkes, Bettina, Dr., Chestnut Hill, MA 02167 (US); Jochner, Nicola Maria, 85354 Freising (DE)
(74) Vertreter: Behnisch, Werner, Dr.

(56) Entgegenhaltungen:
- EMBO J., - 3.Januar 1995 XP002023504 KEMPKES B. ET AL.,: "B-cell proliferation and induction of early G1-regulating proteins by Epstein-Barr virus mutants conditional for EBNA2"
- INT. J. CANCER, Bd. 39, - 15.März 1987 Seiten 404-408, XP000616321 WENDEL-HANSEN V. ET AL.,: "EBV-transformed lymphoblastoid cell lines down-regulated EBNA in parallel with secretory differentiation"
- EUR. J. IMMUNOL., - Juni 9195 XP000616296 LARCHER C. ET AL.,: "Expression of Epstein-Barr virus nuclear antigen-2 (EBNA2) induces CD21/CR2 on B and T cell lines and shedding of soluble CD21"
- EMBO J. , Bd. 15, Nr. 2, - 15.Januar 1996 Seiten 375-382, XP002023503 JOCHNER N. ET AL., : "Epstein-Barr virus nuclear antigen 2 is a transcriptional suppressor of the immunoglobulin u gene: implications for the expression of the translocated c-myc gene in Burkitt's lymphoma cells"

## Beschreibung

Die Erfindung betrifft ein Verfahren zur Herstellung von menschlichen monoklonalen Antikörpern nach dem Oberbegriff von Patentanspruch 1.

Antikörper finden eine sehr breite Anwendung in der Behandlung und Therapie von Menschen. Antikörper werden verwendet in der Immunprophylaxe, bei der Behandlung akuter Infektionen sowie bei der Behandlung immunsupprimierter Patienten. Menschliche Antikörper sind heute nicht in ausreichender Menge herstellbar. Ein Verfahren der gattungsgemäßen Art ist z.B. aus Casali et al., (1986), Science 234, 476-479, bekannt.

Es werden deswegen häufig tierische Antikörper eingesetzt. Tierische Antikörper haben den Nachteil, daß sie beim Menschen störende Abwehrreaktionen hervorrufen und ihre Wirkung verloren geht. Menschliche Antikörper kann man aus menschlichem Serum gewinnen: Dieses Verfahren hat drei wesentliche Nachteile: Geeignetes menschliches Serum steht nur in begrenztem Umfang zur Verfügung, die gereinigten Antikörper sind immer polyklonal, die Behandlung ist sehr teuer.

Aufgabe der Erfindung ist es, ein Verfahren der eingangs genannten Art so auszugestalten, daß monoklonale, menschliche Antikörper in ausreichender Menge und hoher Spezifität hergestellt werden können.

Diese Aufgabe wird durch die kennzeichnenden Merkmale des Patentanspruchs 1 gelöst. Die Unteransprüche beschreiben vorteilhafte Ausgestaltungen des Verfahrens. Die durch das erfindungsgemäße Verfahren hergestellten Antikörper werden bevorzugt in einer pharmazeutischen Zubereitung als Therapeutika eingesetzt.

Das Verfahren bietet zum ersten Mal die Möglichkeit, menschliche Antikörper in der Zellkultur in solchen Mengen herzustellen, daß eine Reinigung der Antikörper und deren medizinische Anwendung möglich ist.

Nachfolgend wird die Erfindung anhand bevorzugter Ausgestaltungen näher erläutert; die Erfindung ist jedoch nicht hierauf beschränkt, sondern kann in Rahmen der Patentansprüche variiert werden. Weitere Ausführungsformen sind vom Fachmann ohne erfinderisches Zutun durchführbar.

Erfindungsgemäß werden somit zunächst Antikörper produzierende B-Zellen mit einem Epstein-Barr-Virus oder einem entweder natürlich vorkommenden oder gentechnisch hergestellten Derivat des Epstein-Barr-Virus infiziert. In einer weiteren Ausführungsform der Erfindung wird ein Vektor verwendet, der zumindest diejenigen Sequenzen des Epstein-Barr-Virus aufweist, die zur Immortalisierung menschlicher B-Zellen notwendig sind. Bevorzugt werden primäre menschliche B-Zellen infiziert.

Epstein-Barr-Virus (EBV) ist ein lymphotropes Herpes-Virus, das für die infektiöse Mononukleose verantwortlich ist. Die infektiöse Mononukleose, auch Pfeiffer-Drüsenfieber genannt, ist eine lymphoproliferative Erkrankung, die zu einer Hyperplasie und Hypertrophie des lymphatischen Gewebes mit charakteristischen Blutbildveränderungen führt. EBV infiziert primäre, ruhende B-Zellen, wodurch in vitro unbegrenzt proliferierende lymphoblastoide Zellinien (LCLs) entstehen. Dieser Prozeß wird auch als Immortalisierung oder Transformation bezeichnet. EBV wird weiterhin mit verschiedenen menschlichen Erkrankungen assoziiert, zu denen das Burkitt-Lymphom (BL), Nasopharynx-Karzinome, Lymphome immunkompromittierter Personen und Morbus Hodgkin (vgl. Übersichten in Miller, 1990; Klein, 1994; Masucci und Ernberg, 1994) gehören.

In LCL-Zellen wird nur ein Teil der viralen Gene exprimiert. Die exprimierten Virusgene kodieren für sechs Kernproteine, drei Membranproteine und zwei kleine Kern-RNAs, die nicht polyadenyliert sind. Die minimale Anzahl von Genen, die für die Transformation erforderlich ist, ist bis heute unbekannt. Das EBNA2-Gen ist in dem transformationsdefekten Virus, der von der Burkitt-Lymphomzellinie P3HR1 produziert wird, deletiert. Es kommt in natürlichen Isolaten in zwei allelen Formen vor (EBNA2A und EBNA2B), die in etwa 50% Homologie zeigen (Zimber et al., 1986). EBNA2 ist zusammen mit EBNA-LP das erste Virusgen, das nach Infektion primärer B-Zellen exprimiert wird, und es ist ein Transkriptionsaktivator sowohl von latenten Virusals auch von latenten Zellgenen (CD21, CD23 und c-fgr) (Calender et al., 1987; Wang et al., 1987; Cordier et al., 1990; Ghosh und Kieff, 1990; Fahraeus et al., 1990; Abbot et al., 1990; Knutson, 1990; Woisetschlaeger et al., 1991; Zimber-Strobl et al., 1991, 1993; Sung et al., 1991; Jin und Speck, 1992; Ling et al., 1993; Laux et al., 1994a; Meitinger et al., 1995).

Es konnte gezeigt werden, daß EBNA2 seine Transaktivierungsfunktion zumindest teilweise durch Bindung an ein ubiquitär exprimiertes Zellgen, RBP-Jκ, das an die DNA in sequenzspezifischer Weise bindet (Zimber-Strobl et al., 1994; Henkel et al., 1994; Grossmann et al., 1994; Waltzer et al, 1994) und durch Wechselwirkung mit Transkriptionsfaktorender ets-Genfamilie (SPi-1, PU-1) (Laux et al., 1994b; und Johannsen et al., 1995) bewirkt. Obwohl die Transformation primärer B-Zellen in vitro strikt von EBNA2 abhängt (Cohen et al., 1989; Hammerschmidt und Sugden, 1989; Kempkes et al., 1995), scheint EBNA2 in BL-Tumoren (Rowe et al., 1986), beim Morbus Hodgkin (Kanavaros et al., 1993) und in Nasopharynx-Karzinomen (Fahraeus et al., 1988; Young et al., 1988) nicht exprimiert zu werden, weshalb seine Rolle bei der Entstehung dieser Erkrankungen in vivo unklar ist.

Um die Rolle von EBNA2 bei der Transformation von B-Zellen besser zu verstehen, wurde ein konditionales System entwickelt, bei dem die Funktion des EBNA2-Proteins reversibel an- und ausschaltbar ist. Unter Verwendung des von Picard et al. (1988) und Eilers et al. (1989) entwickelten induzierbaren Systems wurde die Hormonbindungsdomäne des Östrogenrezeptors mit dem N- oder C-Terminus von EBNA2 fusioniert, wodurch die Funktion des EBNA2-Proteins nunmehr davon abhängt, ob Östrogen vorhanden ist oder nicht. Wie erwartet, bewirken die chimeren EBNA2-Proteine ihre transaktivierende Funktion auf virale und zelluläre Gene nur in Gegenwart von Östrogen.

Erfindungsgemäß wurde überraschend gefunden, daß durch das EBNA2-Protein die Expression von Oberflächen-IgM und die Transkription des Ig-µ-Locus sehr effizient verringert wird. EBNA2 wirkt somit als negativer Regulator der Ig-µ-Transkription. Die Erfindung wird im folgenden anhand von Ausführungsbeispielen mit Hilfe der Figuren näher erläutert. Dabei zeigen die Figuren 1 und 2 exemplarisch die Erhöhung der Immunglobulinprotein-Produktion durch Abschaltung von EBNA2. Die Figuren 3 und 4 zeigen entsprechend die Immunglobulin-RNA-Produktion.

Die Erfindung beruht auf der überraschenden Tatsache, daß Epstein-Barr-Virus-infizierte B-Zellen nur geringe Mengen Immunglobulin produzieren, die Produktion nach Inaktivierung des EBNA2-Gens aber um das mindestens 100-fache steigt.

Die Erfindung wird in den Ausführungsbeispielen anhand einer IgM-produzierenden B-Zellinie näher beschrieben. Die Erfindung ist jedoch nicht auf eine derartige, IgM-produzierende B-Zellinie beschränkt. Erfindungsgemäß können auch B-Zellen durch EBV immortalisiert werden, die andere Immonglobulinklassen exprimieren, beispielsweise IgG und IgA.

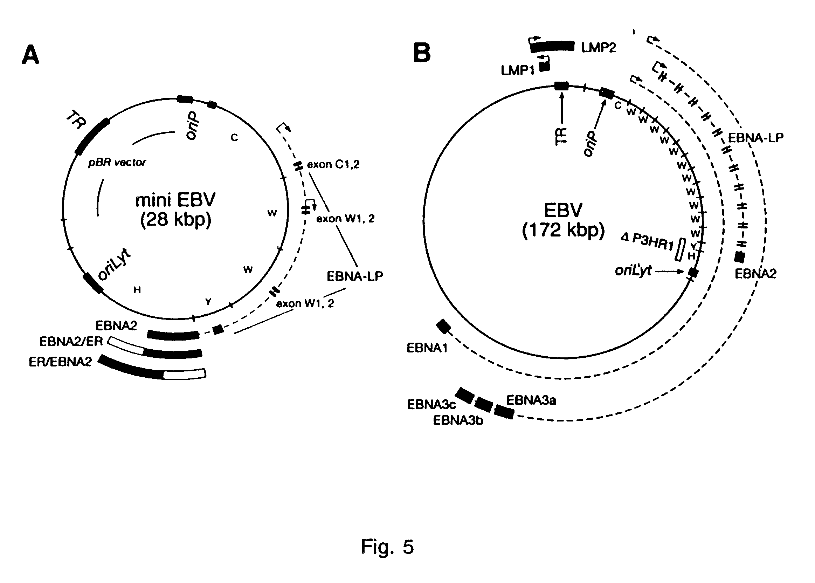

Ursprung der verwendeten B-Zellen sind im Normalfall Menschen mit einem hohen Antikörpertiter für das gewünschte Antigen. Zellen werden vorzugsweise aus dem peripheren Blut gewonnen, und B-Zellen werden nach Standardverfahren angereichert und mit einem Epstein-Barr-Virus-abgeleiteten Virus, welches ein konditionales EBNA2 exprimiert, infiziert. Während der Infektion und Vermehrung der B-Zellen muß die EBNA2-Funktion aktiviert sein. Das von Kempkes et al. (1995) EMBO J. 14, 88-96, beschriebene Virus ist zur Infektion besonders geeignet (vgl. Fig. 5) und die nachfolgende Beschreibung. Zur Aktivierung der EBNA2-Funktion muß Östrogen zum Kulturmedium gegeben werden, um eine konstitutive EBNA2-Funktion zu gewährleisten. Die immortalisierten B-Zellen werden expandiert und mehrmals mit östrogenhaltigem Medium verdünnt, bis die gewünschte Zellzahl erreicht ist. Die Zellen werden in Mikrotiterplatten in geringer Zellzahl ausplattiert und erneut expandiert. Der Überstand der Zellen wird auf die gewünschten spezifischen Antikörper hin untersucht. B-Zellen, die Antikörper mit hoher Spezifität produzieren, werden als Grundlage der Antikörperherstellung verwendet.

In diesen B-Zellklonen wird die EBNA2-Aktivität durch Auswaschen des Östrogens abgeschaltet. Andere Methoden der Entfernung des Östrogens sind ebenfalls anwendbar. Alternativ kann die Aktivität des EBNA2-Gens auch mit der Hormonbindungsdomäne des Androgenrezeptors oder einer anderen Hormonbindungsdomäne oder auf transkriptioneller Ebene durch Verwendung konditionaler Promotoren für das EBNA2-Gen reguliert werden. Mit Abschaltung der EBNA2-Funktion wird die Immunglobulin-Produktion in den B-Zellen kontinuierlich gesteigert und ist nach 2 Tagen ca. 100-fach angestiegen. Nach 4 Tagen beginnt ein großer Teil der Zellen zu sterben.

Nachfolgend wird die Konstruktion eines Plasmids beschrieben, das zur konditionalen Expression des EBNA2-Gens oder einem Derivat hiervon verwendbar ist. Die Erfindung ist jedoch nicht auf das hier beschriebene Plasmid beschränkt. Erfindungsgemäß können auch andere Vektoren verwendet werden, mit denen das EBNA2-Gen konditional exprimierbar ist, d.h. bei der Immortalisierung der B-Zellen zumindest teilweise angeschaltet ist und vor Gewinnung der Antikörper zumindest teilweise abgeschaltet ist. Bevorzugt ist das EBNA2-Gen zur Immortalisierung im wesentlichen angeschaltet und zur Gewinnung der Antikörper im wesentlichen abgeschaltet.

Die Hormonbindungsdomäne des Östrogenrezeptors wurde entweder an den N- oder C-Terminus des offenen Leserahmens (open reading frame = ORF) von EBNA2 angehängt, wodurch die Plasmide ER/EBNA2 und EBNA2/ER entstanden. Beide Plasmide wiesen die Gesamtstruktur des Mini-EBV p554 auf und exprimierten Wildtyp-EBNA2, EBNA-LP und den offenen Leserahmen vom BHRF1 (Hammerschmidt und Sugden, 1989). Das N-terminale Fusionskonstrukt wurde durch Einführung einer EcoRI-Schnittstelle unmittelbar 5' an den ORF von EBNA2 durch in vitro-Mutagenese gebildet. Das für die Hormonbindungsdomäne kodierende Genfragment wurde durch die Polymerasekettenreaktion (PCR) unter Verwendung von HE14 (Kumar et al., 1986) als Matrize, flankiert von den EcoRI-Erkennungstellen und ligiert in die EcoRI-Erkennungsstelle vor den ORF von EBNA2, gebildet. Dieses PCR-Fragment umfaßte die von HE14 bereitgestellte Kozak-Sequenz, jedoch nicht das Stop-Codon des Östrogenrezeptors. Das C-terminale Fusionskonstrukt wurde durch Insertion von BglII- und EcoRI-Erkennungsstellen unmittelbar von dem Stop-Codon von EBNA2 durch in vitro-Mutagenese erzeugt. In die BglII/EcoRI-Erkennungsstellen am EBNA2-Ende wurde ein BamHI-EcoRI-Fragment von HE14, das für die Hormonbindungsstelle des Östrogenrezeptors kodiert, ligiert. Die nachfolgenden Klonierungsschritte ergaben den EBV-Stamm B95.8 mit den Koordinaten 13944-54364 (vgl. Baer et al., 1984) mit zwei Abänderungen: die NotI-Repeats waren deletiert und nur zwei BamHI W-Repeats waren insertiert.

Die Fig. 5 zeigt die Strategie zur Expression konditionaler EBNA2-Mutanten in wachstumstransformierten B-Zellen.

(A) der Fig. 5 zeigt Mini-EBV-Plasmide, die aus in cis wirkenden Elementen bestehen, die zur Vermehrung dieser Plasmide in allen Phasen des Lebenszyklus des Epstein-Barr-Virus essentiell sind. Diese Mini-EBV-Plasmide tragen den Replikationsursprung des Plasmids (oriP), der für die Beibehaltung der EBV-Plasmide in den Targetzellen notwendig ist, den lytischen Replikationsursprung (oriLyt), der bei Induktion der lytischen Replikation des Virus eine Amplifikation mit hoher Kopienanzahl ermöglicht, und die terminalen Repeats (TR), die eine Verpackung der Mini-EBV-Plasmide in Virionen ermöglichen. Die Hormonbindungsdomäne des Östrogenrezeptors (weiße Kästchen) wurden entweder an den C- oder N-Terminus des offenen Leserahmens des Wildtyp-EBNA2 (schwarze Kästchen) angehängt, wodurch die EBNA2/ER- bzw. ER/EBNA2-Mutanten entstanden. Die EBNA2-Expressionskassetten werden von EBV-Sequenzen flankiert, den BamHI-Restriktionsfragmenten C, W, Y und H des EBV-Genoms, die teilweise die Genomorganisation der EBV-DNA darstellen und die die im nicht-transformierenden P3HR1-EBV-Stamm vorhandene Deletion umfassen (ΔP3HR1, offener Balken in B). EBNA2 und EBNA2-Mutanten können von zwei alternativen Promotoren transkribiert werden, die in den C- oder W-Fragmenten lokalisiert sind.

Die Fig. 5 (B) zeigt die EBV-Karte mit den in wachstumstransformierten B-Zellen transkribierten EBNAs und LMPs und die Lokalisierung ihrer Promotoren in bezug auf die BamHI-Restriktionskarte des EBV-Stammes B95.8.

Bei den östrogenabhängigen Zellinien ER/EB2-3 und ER/EB2-5 handelt es sich um lymphoblastoide Zellinien (LCLs), die durch Coinfektion primärer B-Zellen mit P3HR1-Virus und einem Mini-EBV-Plasmid, das ER/EBNA2 exprimiert und das den EBNA2-Defekt des P3HR1-Virus in trans komplementiert, etabliert wurden (Kempkes et al., 1995). Die östrogenunabhängige EB2-2-Zellinie wurde parallel hierzu unter Verwendung von Wildtyp-EBNA2 erzeugt, um den EBNA2-Defekt des P3HR1-Virus zu komplementieren. BL41 ist eine EBV-negative BL-Zellinie mit einer t(8;14)-Translokation (Lenoir et al., 1985), BL41/P3HR1 und BL41/B95-8 sind BL41-Zellen, die mit P3HR1- bzw. B95-8-Virus stabil infiziert (umgewandelt) wurden (Calender et al, 1987). BJAB ist eine EBV-negative B-Lymphomzellinie (Klein et al, 1974); BJAB/P3 ist eine BJAB-Zellinie, die mit P3HR1-Virus stabil infiziert ist. P3HR1 ist ein Einzelzellklon der EBV-positiven B-Zellinie Jijoye mit einer t(8;14)-Translokation (Hinuma et al, 1967). Jijoye produziert ein EBNA2-kompetentes und die P3HR1-Zellen ein EBNA2-defektes Virus (Bornkamm et al., 1982). Die ER/EBNA2 exprimierenden Lymphomzellinien sind bei Kempkes et al. beschrieben. Alle Zellinien wurden in RPMI1640-Medium kultiviert, das mit 10% fötalem Kälberserum, 100 U/ml Penicillin und Streptomycin und 1 mM Pyruvat supplementiert war. β-Östradiol (Merck, Darmstadt) wurde zum Zellkulturmedium in einer Endkonzentration von 1 µM zugegeben.

Zur Durchführung der FACS-Analyse wurden 1 x 10⁶ Zellen mit einem Überschuß an unmarkierten monoklonalen Antikörpern der Maus, die CD21 (BL13) und Oberflächen-IgM (AF6) (Dianova, Hamburg) erkennen, inkubiert. Mit FITC (Fluorescein) konjugierte Ziege-anti-Maus F(ab)₂-Fragmente (DAKO, Hamburg) wurden zur Färbung positiver Zellen als sekundärer Antikörper verwendet. Tote Zellen wurden identifiziert und nach Propidiumiodidfärbung (0,1 µg/ml) von der Analyse ausgeschlossen. Die Proben wurden unter Verwendung eines FACSSCAN-Geräts (Becton und Dickinson) analysiert. Der 3-H-Thymidineinbau-Test wurde wie bei Kempkes et al., 1995 beschrieben durchgeführt.

Die Northern-Blot-Analysen wurden wie bei Eick und Bornkamm, 1989 beschrieben durchgeführt. Als Igµ-Gen-Sonden wurde das 1,2 kb große EcoRI/EcoRI-Fragment und das 0,9 kb EcoRI/SacI-Fragment der konstanten Region, beschrieben in Eick et al. (1985), verwendet.

In den oben beschriebenen, konditional transformierten lymphoblastoiden Zellen wurde die Expression von Oberflächenmarkern vor und nach Entfernung von Östrogen untersucht.

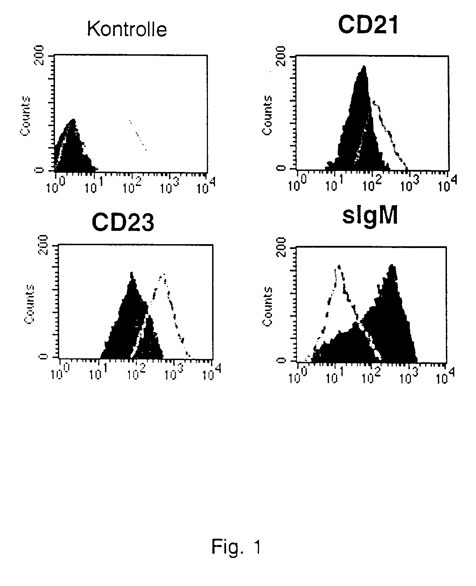

Die Fig. 1 zeigt eine Flußzytometrieanalyse von mit Östrogen behandelten (weiße Histogramme) und an Östrogen verarmten (schwarze Histrogramme) ER/EB2-5-Zellen nach CD21-, CD23- und Oberflächen-IgM-Färbung.

Die Fig. 1 zeigt insbesondere die Menge an IgM-Antikörpern, die in Anwesenheit (weiße Fläche) und Abwesenheit (schwarze Fläche) von EBNA2-Aktivität von einer EBV-immortalisierten B-Zelle nach 2 Tagen produziert wird. Vergleichbare Ergebnisse wurden nach Immortalisierung primärer B-Zellen mit dem oben genannten Virus für zwei weitere Zellinien erhalten.

Die Fig. 1 zeigt, daß die CD23- und in einem geringeren Ausmaß die CD21-Expression durch Abschaltung der Funktion von EBNA2 durch Entfernung des Östrogens abnahm. Gleichzeitig nahm jedoch die IgM-Expression stark zu. Die Zunahme der IgM-Expression und die Abnahme der CD21- und CD23-Expression waren reversibel, da sowohl die CD21- als auch die CD-23-Expression zunahmen und die IgM-Expression abnahm, wenn anschließend Östrogen zugegeben wurde. Die Ergebnisse sind hier nicht im einzelnen wiedergegeben.

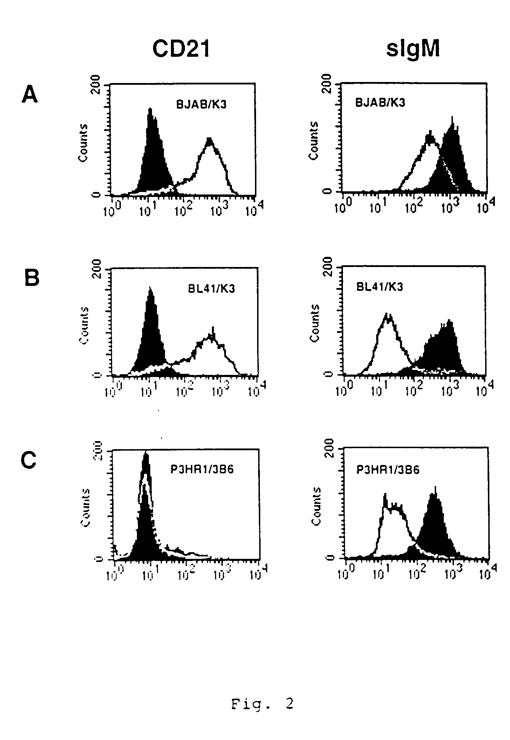

Die Abnahme des Oberflächen-IgMs könnte entweder durch EBNA2 selbst oder durch andere virale, stromabwärts gelegene Targets (z.B. die viralen latenten Membranproteine), die durch EBNA2 induziert werden, vermittelt werden. Um zwischen diesen zwei Möglichkeiten zu unterscheiden, wurde das das ER/EBNA2-Fusionsprotein kodierende Gen stabil in die EBV-negativen B-Zell-Lymphomlinien BL41 und BJAB und zum Vergleich auch in P3HR1-Zellen eingeführt. In den stabil transfizierten Zellinien war die Aktivierung des TP1- oder des LMP1-Promotors strikt an das Vorliegen von Östrogen gebunden, was zeigt, daß das ER/ENBA2-Protein tatsächlich in diesen Zellen funktionell ist. Eine FACS-Analyse vor und nach Zugabe von Östrogen zeigte eine starke Abnahme der sIgM-Expression bei Zugabe von Östrogen (Fig. 2).

Hieraus kann geschlossen werden, daß die Abnahme der sIgM-Expression eine Funktion von EBNA2 ist und keine Funktion eines anderen viralen Targetgens.

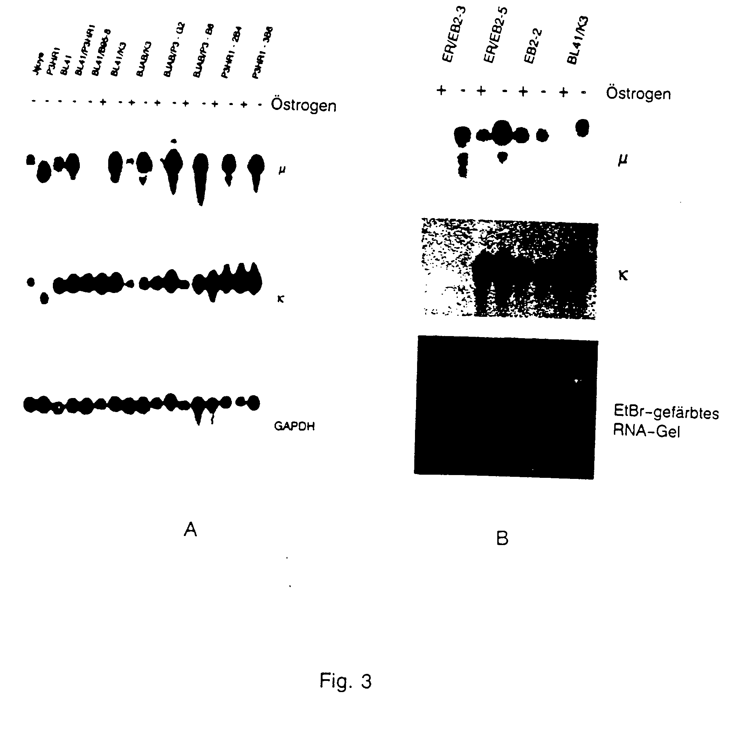

Es wurden Northern-Blot-Analysen durchgeführt, um herauszufinden, bei welchem Niveau die IgM-Expression durch EBNA2 nach unten reguliert wird. Durch die Aufnahme von RNA aus Paarzellinien, die sich durch das Vorliegen oder das Fehlen des Wildtyp EBNA2-Gens unterscheiden, ergab die Northern-Blot-Analyse weiterhin eine Antwort auf die Frage, ob die IgM-Abnahme nicht nur durch das EBNA2-Östrogen-Rezeptorfusionsprotein bewirkt wird, sondern auch durch das Wildtyp-EBNA2. Die Paarzellinien umfaßten P3HR1, das ein EBNA2-defekter Virus produziert, und seine Elternzellinie Jijoye mit einem funktionellen EBNA2-Gen (EBNA2B-Allel) und BL41-Zellen, die mit P3HR1-Virus stabil infiziert/umgewandelt (konvertiert) waren (BL41/p3HR1) oder dem transformationskompetenten, EBNA2-Wildtyp-positiven B95-8-Virus mit dem EBNA2-Allel. Wie die Fig. 3 zeigt, wurde die Oberflächen-Expression durch EBNA2 durch Abnahme der Igµ-RNA nach unten reguliert.

Die Figur 2 zeigt die Menge an IgM-Antikörpern, die in Anwesenheit (weiße Fläche) und Abwesenheit (schwarze Fläche) von ENBA2-Aktivität von zwei Burkitt-Lymhom-Zellinien nach 2 Tagen produziert wird.

Die Fig. 2 zeigt insbesondere die Suppression von Zelloberflächen-IgM durch EBNA2 in B-Lymphomzellinien. ER/EBNA2-transfizierte Zellen (BJAB/K3, BL41/K3 und P3HR1/3B6) wurden 2 Tage lang in Gegenwart von (weiße Histogramme) oder ohne (schwarze Histogramme) 1 µM β-Östradiol kultiviert und dann auf Zelloberflächen-CD21 und Oberflächen-IgM-Expression durch FACS-Analyse untersucht. 1 x 10⁶ Zellen wurden mit einem Überschuß an unmarkierten monoklonalen Antikörpern der Maus inkubiert, die Human-CD21 (BL13) oder IgM (AF6) erkennen. FITC-konjugierte Ziege-anti-Maus F(ab)₂-Fragmente wurden als sekundärer Antikörper zur Färbung von positiven Zellen verwendet. Kontrollzellen wurden nur mit sekundären Antikörpern markiert; sie zeigten jedoch keinerlei Veränderungen auf Östrogenzugabe (Daten nicht gezeigt). Tote Zellen wurden identifiziert und aus der Analyse nach Propidiumiodidfärbung (=,1 µg/ml) ausgeschlossen.

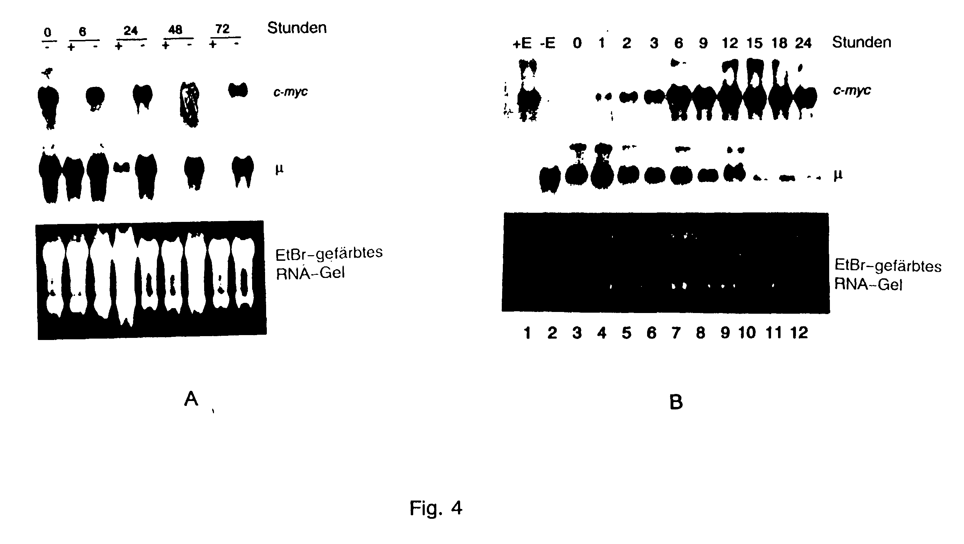

Die Figuren 3 und 4 zeigen in analoger Weise die Menge der entsprechenden Immunglobulin-RNAs.

Die Fig. 3 zeigt speziell die Suppression der Expression der schweren Kette von Ig-µ durch EBNA2 in B-Lymphomzellen und LCLs. Gesamtzell-RNA wurde aus JiJoye, P3HR1, BL41, BL41/P3HR1, BL41/B95-8 und ER/ENBA2-transfizierten Zellen, die zwei Tage lang mit oder ohne Östrogene (A) kultiviert worden waren, oder aus LCL-Zellinien, die das ER/EBNA2-Protein (ER/EB2-3 und ER/EB2-5) oder das Wildtyp EBNA2-Protein (EB2-2) exprimieren, wobei die Zellinien mit Östrogen behandelt worden waren oder vier Tage lang an Östrogen verarmt wurden (B), isoliert, und die Zellen wurden in bezug auf die schwere Kette von Ig-µ-, die leichte Kette von Ig-κ und GAPDH-RNA durch Northern-Blot-Analyse analysiert.

Die Fig. 4 zeigt die Coregulation der Ig-µ- und der c-myc-Gen-expression in ER/EBNA2-transfizierten BL-Zellen. P3HR1/3B6 (A)-Zellen wurden mit Östrogen (+) für die angegebene Zeitdauer behandelt und auf Expression von Ig-µ- und c-myc-RNA durch Northern-Analyse untersucht. Am unteren Rand des Bildes sind die mit Ethidiumbromid gefärbten Gele gezeigt. In östrogenabhängigen LCLs werden die IG-µ- und die c-myc-Expression durch Östrogen einander entgegengesetzt reguliert (B).

Durch das erfindungsgemäße Verfahren können somit zum ersten Mal menschliche Antikörper aus B-Zellen in großen Mengen bereitgestellt werden. Hierzu werden mit EBV oder einem EBV-Derivat immortalisierte B-Zellen verwendet, die das EBNA2-Gen in konditionaler Form enthalten. Zur Immortalisierung der B-Zellen wird das EBNA2-Gen angeschaltet und zur Produktion der Antikörper wird das EBNA2-Gen abgeschaltet.

Die Überstände von Zellkulturen werden bevorzugt zwischen Tag 2 und 5 geerntet und die Antikörper gereinigt. Diese stehen dann zu therapeutischen Zwecken zur Verfügung. Verfahren zur Gewinnung der Antikörper sind aus dem Stand der Technik bekannt und können vom Fachmann auf das vorliegende Verfahren ohne weiteres übertragen werden.

Die Antikörper werden vorzugsweise dort verwendet, wo tierische oder manipulierte tierische Antikörper zu unerwünschten Nebenwirkungen beim Menschen führen.

Erfindungsgemäß wird somit ein Verfahren zur Herstellung menschlicher monoklonaler Antikörper bereitgestellt, daß die nachfolgenden Schritte aufweist:
a) Infizieren oder transfizieren menschlicher, Antikörper produzierender B-Zellen mit einem Epstein-Barr-Virus oder einem Derivat dieses Virus in einem ersten Kulturmedium, wobei das Virus oder das Derivat ein EBNA2-Protein expremiert, das im ersten Kulturmedium funktionell ist, um immortalisierte B-Zellen herzustellen;
b) Screenen der immortalisierten B-Zellen auf ein gewünschtes Antigen, um einen immortalisierten B-Zell-Klon zu identifizieren, der Antikörper produziert, die für das gewünschte Antigen spezifisch sind; und
c) Expandieren des identifizierten B-Zell-Klons im ersten Kulturmedium;
d) Kultivieren des expandierten B-Zell-Klons in einem zweiten Kulturmedium, wobei die Expression oder Funktion des EBNA2-Proteins zumindest verringert wird und die Antikörperproduktion hierdurch erhöht wird;
e) Wiedergewinnen der Antikörper aus dem zweiten Kulturmedium.

Erfindungsgemäß kann das Derivat des Epstein-Barr-Virus auch ein Mini-EBV-Virus sein, das zusammen mit einem Helfervirus eingesetzt wird.

Das EBV-Virus oder sein Derivat weist in einer bevorzugten Ausführungsform der Erfindung eine Nukleotidsequenz auf, die für EBNA2 kodiert und die, in funktioneller Verbindung, mit einer Nukleotidsequenz steht, die für eine Hormon-Bindungsdomäne kodiert, so daß ein Fusionsgen hergestellt wird, das für ein Fusionsprotein kodiert, das EBNA2 und die Hormon-Bindungsdomäne umfaßt. Die Hormon-Bindungsdomäne bindet bevorzugt Östrogen oder ein Androgen.

Das oben genannte erste Kulturmedium umfaßt ein Hormon, das für die Hormon-Bindungsdomäne spezifisch ist, und zwar in einer Konzentration, die ausreichend ist, um immortalisierte B-Zellen zu produzieren, und das oben genannte zweite Kulturmedium weist eine ausreichend verringerte Konzentration des Hormons im Vergleich zum ersten Kulturmedium auf, um die Antikörperproduktion durch den expandierten B-Zell-Klon zu erhöhen.

In einer weiteren Ausführungsform der Erfindung weist das Virus oder sein Derivat eine Nukleotidsequenz auf, die für EBNA2 kodiert und die in funktioneller Verbindung mit einer Nukleotidsequenz steht, die eine induzierbare Expression der für EBNA2 kodierenden Nukleotidsequenz gestattet.

Das EBNA2-Protein wird somit bevorzugt als Fusionsprotein mit der Hormon-Bindungsdomäne des Östrogenrezeptors exprimiert oder das EBNA2-Protein steht unter Kontrolle eines induzierbaren Promoters. Das Virus oder das Derivat ist bevorzugt ein sich vom Epstein-Barr-Virus ableitender Vektor, der diejenigen Epstein-Barr-Virussequenzen trägt, die zur Immortalisierung notwendig sind. Die Expression des EBNA2-Proteins kann somit erfindungsgemäß unter Kontrolle eines induzierbaren Promoters stehen, wobei die Expression durch einen Induktor im Schritt a) induziert wird und durch Entfernen des Induktors vor dem Schritt d) wieder verringert wird.

### Literatur

Abbot, S.D., Rowe, M., Cadwallader, K., Ricksten, A., Gordon, J., Wang, F., Rymo, L. and Rickinson, A.B. (1990). Epstein-Barr virus nuclear antigen 2 induces expression of the virus encoded latent membrane protein. *J. Virol.* **64,** 2126-2134.

Albert, T., Urlbauer, B., Kohlhuber, F., Hammersen, B. and Eick, D. (1994). Ongoing mutations in the N-terminal domain of c-Myc affect transactivation in Burkitt's lymphoma cell lines.*Oncogene* **9,** 759-763

Allen, R.W., Trach, K.A. and Hoch, J.A. (1987). Identification of the 37-kDa protein displaying a variable interaction with the erythroid cell membrane as glyceraldehyde-3-phophate dehydrogenase. *J. Biol. Chem.* **262,** 649-653.

Benjamin, D., Magrath, I.T., Maguire, R., Janus, C., Todd, H.D. and Parsons, R.G. (1982). Immunoglobulin secretion by cell lines derived from african and american undifferentiated lymphomas of Burkitt's and non-Burkitt's type. *J. Immunol.* **129,** 1336-1342.

Bornkamm, G.W., Hudewentz, J., Freese, U.K. and Zimber, U. (1982). Deletion of the nontransforming Epstein-Barr virus strain P3HR-1 causes fusion of the large internal repeat to the DSL region. *J. Virol.* **43,** 952-968.

Bornkamm, .W., Polack, A. and Eick, D. (1988). In Klein, G. (ed.), Cellular oncogene activation; c-*myc* Deregulation by Chromosomal Translocation in Burkitt's Lymphoma. M. Dekker, Inc., New York, Basel, pp 223-273.

Calender, A., Billaud, M., Aubry, J.P., Banchereau, J., Vuillaume, M. and Lenoir, G.M. (1987). Epstein-Barr virus (EBV) induces expression of B-cell activation markers on in vitro infection of EBV-negative B-lymphoma cells. *Proc. Natl. Acad. Sci. U.S.A.* 84, 8060-8064.

Cohen, J.I., Wang, F., Mannick, J. and Kieff, E. (1989). Epstein-Barr virus nuclear protein 2 is a key determinant of lymphocyte transformation. *Proc. Natl. Acad. Sci. U.S.A.* **86,** 9558-9562.

Cohen, J.H.M., Revillard, J.P., Magaud, J.P., Lenoir, G., Vuillaume, M., Manel, A.M., Vincent, C. and Bryon, P.A. (1987). B-cell maturation stages of Burkitt's lymphoma cell lines according to Epstein-Barr virus status and type of chromosome translocation. J. Natl. Cancer Inst. **78**, 235-242.

Cordier Bussat, M., Billaud, M., Calender, A. and Lenoir, G.M. (1993). Epstein-Barr virus (EBV) nuclear-antigen-2-induced up-regulation of CD21 and CD23 molecules is dependent on a permissive cellular context. *Int. J. Cancer* **53,** 153-160.

Cordier, M., Calender, A., Billaud, M., Zimber, U., Rousselet, G., Pavlish, O., Banchereau, J., Tursz, T., Bornkamm, G. and Lenoir, G.M. (1990). Stable transfection of Epstein-Barr virus (EBV) nuclear antigen 2 in lymphoma cells containing the EBV P3HR1 genome induces expression of B-cell activation molecules CD21 and CD23. *J. Virol.* **64,** 1002-1013.

Crawford, D.H. and Ando, I. (1986). EB virus is associated with B-cell maturation. *Immunology* **59,** 405-409.

Dani, C., Blanchard, J.M., Piechaczyk, M., El Sabouty, S., Marty, L. and Jeanteur, J. (1984). Extreme instability of *myc* mRNA in normal and transformed human cells. *Proc. Natl. Acad. Sci. U.S.A.* **81,** 7046-7050.

Eick, D., Piechaczyk, M., Henglein, B., Blanchard, J.-M., Traub, B., Kofler, E., Wiest, S., Lenoir G.M. and Bornkamm, G.W. (1985). Aberrant c-*myc* RNAs of Burkitt's lymphoma cells have longer half lives. *EMBO J.* **4,** 3717-3725.

Eick, D. and Bornkamm, G.W. (1989). Expression of normal and translocated c-*myc* alleles in Burkitt's lymphoma cells: evidence for different regulation. *EMBO J.* **8,** 1965-1972.

Eilers, M., Picard, D., Yamamoto, K.R. and Bishop, J.M. (1989). Chimaeras of *myc* oncoprotein and steroid receptors cause hormone-dependent transformation of cells. *Nature* **340,** 66-68.

Fahraeus, R., Fu, H.L., Ernberg, I., Finke, J., Rowe, M., Klein, G., Falk, K., Nilsson, E., Yadav, M., Busson, P., Tursz, T. and Kallin, B. (1988). Expression of Epstein-Barr virus-encoded proteins in nasopharyngeal carcinoma. *Int. J. Cancer* **42,** 329-338.

Fahraeus, R., Jansson, A., Ricksten, A., Sjoblom, A. and Rymo, L. (1990). Epstein-Barr virus encoded nuclear antigen 2 activates the viral latent membrane protein promoter by modulating the activity of a negative regulatory element. *Proc. Natl. Acad. Sci. U.S.A.* **87,** 7390-7394.

Ghosh, D. and Kieff, E. (1990). Cis-acting regulatory elements near the Epstein-Barr virus latent-infection membrane protein transcriptional start site. *J. Virol.* **64,** 1855-1858.

Grossman, S.R., Johannsen, E., Tong, X., Yalamanchili, R. and Kieff, E. (1994). The Epstein-Barr virus nuclear antigen 2 transactivator is directed to response elements by the Jκ recombination signal binding protein. *Proc. Natl. Acad. Sci. USA* **91,** 7568-7572.

Hammerschmidt, W. and Sugden, B. (1989). Genetic analysis of immortalizing functions of Epstein-Barr virus in human B lymphocytes. *Nature* **340,** 393-397.

Henkel, T., Ling, P.D., Hayward, S.D. and Peterson, M.G. (1994). Mediation of Epstein-Barr Virus EBNA2 Transactivation of by Recombination Signal-Binding protein Jκ. *Science* **265**, 92-95.

Hieter, P.A., Max, E.E., Seidman, J.G., Maizel, J.V. Jr. and Leder, P. (1980). Cloned human and mouse kappa immunoglobulin constant and J region genes conserve homology in functional segments. *Cell* **22,** 197-207.

Hinuma, Y., Konn, M., Yamaguchi, J., Wudarski, D.J., Blakeslee, J.R.,Jr. and Grace, J.T., Jr. (1967). Immunofluorescence and herpes-type virus particles in the P3HR-1 Burkitt lymphoma cell line. *J. Virol.* **1,** 1045-1051.

Jäck, H.M. and Wabl, M. (1988). Immunoglobulin mRNA stability varies during B lymphocyte differentiation. *EMBO J.* **7,** 1041-1046.

Jin, X.W. and Speck, S.H. (1992). Identification of critical cis elements involved in mediating Epstein-Barr virus nuclear antigen 2-dependent activity of an enhancer located upstream of the viral BamHI C promoter. *J. Virol.* **66,** 2846-2852.

Johannsen, E., Koh, E., Mosialos, G., Tong, X., Kieff, E., Grossman, S.R. (1995). Epstein-Barr virus nuclear protein 2 transactivation of the latent membrane protein 1 promoter is mediated by Jκ and PU.1. *J. Virol.* **69,** 253-262.

Kanavaros, P., Jiwa, M., van -der-Valk, P., Walboomers, J., Horstman, A. and Meijer, C.J. (1993). Expression of the Epstein-Barr virus latent gene products and related cellular activation and adhesion molecules in Hodgkin's disease and non-Hodgkin's lymphomas arising in patients without overt pre-existing immunodeficiency. *Hum. Pathol.* **24,** 725-729.

Kaye, K.M., Izumi, K.M. and Kieff, E. (1993). Epstein-Barr virus latent membrane protein 1 is essential for B-lymphocyte growth transformation. *Proc. Natl. Acad.* Sci. *U.S.A.* **90,** 9150-9154.

Kempkes, B., Spitkovsky, D., Jansen-Dürr, P., Ellwart, J., Kremmer, E., Delecluse, H.J., Rottenberger, C., Bornkamm, G.W. and Hammerschmidt, W. (1995). B-cell proliferation and induction of early G1-regulating proteins by Epstein-Barr virus mutants conditional for EBNA2. *EMBO J.*, **14,** 88-95.

Kieff, E. and Liebowitz, D. (1990). Epstein- Barr Virus and Its Replication. In Virology. B.N. Fields, D.N. Knipe, R.M. Chanock, M.S. Hirsch, J.L. Melnick, T.P. Monath, and B. Roizman, eds. (New York: Raven Press), pp. 1889-1920.

Klein, G. (1994). Epstein-Barr virus strategy in normal and neoplastic B cells *Cell* **77,** 791-793.

Klein, G., Lindahl, T., Jondal, M., Leibold, W., Menezes, J., Nilsson, K. and Sundstrom, C. (1974). Continuous lymphoid cell lines with characteristics of B cells (bone-marrow-derived), lacking the Epstein-Barr virus genome and derived from three human lymphomas. *Proc. Natl. Acad. Sci. U.S.A.* **71,** 3283-3286.

Knutson, J.C. (1990). The level of c-fgr RNA is increased by EBNA-2, an Epstein-Barr virus gene required for B-cell immortalization. *J. Virol.* **64,** 2530-2536.

Laux, G., Dugrillon. C., Eckert, B., Adam, B., Zimber-Strobl, U. and Bornkamm, G.W. (1994a). Identification and characterization of an Epstein-Barr virus nuclear antigen 2-responsive element in the bidirectional promoter region of latent membrane protein and terminal protein 2 genes. *J. Virol.* **68,** 6947-6958.

Laux, G., Adam, B., Strobl, L.J. and Moreau-Gachelin, F. (1994b). The Spi-1/PU.1 and Spi-B ets family transcription factors and the recombination signal binding protein RBP-Jκ interact with an Epstein-Barr virus nuclear antigen 2 responsive cis-element. *EMBO J.* **13**, 5624-5632.

Lenoir, G.M., Vuillaume, M. and Bonnardel, C. (1985). *Burkitt's Lymphoma: a Human Cancer Model*. The use of lymphomatous and lymphoblastoid cell lines in the study of Burkitt's lymphoma. IARC. Sci. Publ. 309-318.

Le Roux, A., Kerdiles, B., Walls, D., Dedieu, J.F. and Perricaudet, M. (1994). The Epstein-Barr virus nuclear antigens EBNA-3A, -3B, and -3C repress EBNA-2-mediated transactivation of the viral terminal protein 1 gene promoter. *Virology* **205,** 596-602.

Ling, P.D., Rawlins, D.R. and Hayward, S.D. (1993). The Epstein-Barr virus immortalizing protein EBNA-2 is targeted to DNA by a cellular enhancer-binding protein. *Proc. Natl. Acad. Sci. U.S.A.* **90,** 9237-9241.

Madisen, L. and Groudine, M. (1994). Identification of a locus control region in the immunoglobulin heavy-chain locus that deregulates c-*myc* expression in plasmacytoma and Burkitt's lymphoma cells. *Genes Dev.* **8,** 2212-2226.

Magrath, I.T., Freeman, C.B., Pizzo, P., Gadek, J., Jaffe, E., Santaella, M., Hammer, C., Frank, M., Reaman, G. and Novikovs, L. (1980). Characterization of lymphoma-derived cell lines: comparison of cell lines positive and negative for Epstein-Barr virus nuclear antigen. II. Surface markers. *J. Natl. Cancer Inst.* **64,** 477-483.

Marcu, K.B., Bossone, S,A. and Patel, A.J. (1992). *myc* function and regulation. *Annu. Rev. Biochem.* **61,** 809-860.

Marshall, D. and Sample, C. (1995). Epstein-Barr virus nuclear antigen 3C is a transcriptional regulator. *J. Virol.* **69,** 3624-3630.

Masucci, M.G. and Ernberg, I. (1994). Epstein Barr-virus: adaption to a life within the immune system. *Trends Microbiol*. **2,** 125-130.

Matsunami, N., Hamaguchi, Y., Yamamoto, Y., Kuze, K., Kangawa, K., Matsuo, H., Kawaichi, M. and Honjo, T. (1989). A protein binding to the J kappa recombination sequence of immunoglobulin genes contains a sequence related to the integrase motif. *Nature* **342,** 934-937.

Meitinger, C., Strobl, L.J., Marshall, G., Bornkamm, G.W. and Zimber-Strobl, U. (1994). Crucial sequences within the Epstein-Barr virus TP1 - promoter for EBNA2 mediated transactivation and interaction of EBNA2 with its responsive element. *J. Virol.* **68,** 7497-7506.

Miller, G. (1990). In: Virology. Epstein-Barr virus: biology, pathogenesis, and medical aspects. B.N. Fields, D.N. Knipe, R.M. Chanock, M.S. Hirsch, J.L. Melnick, T.P. Monath, and B. Roizman, eds. (New York: Raven Press), pp. 1921-1958.

Nishikura, K., ar-Rushdi, A., Erikson, J., deJesus, E., Dugan, D. and Croce, C. (1984). Repression of rearranged µ gene and translocated c-*myc* in mouse 3T3 cells x Burkitt lymphoma cell hybrids. *Science* **224,** 399-402.

Picard, D., Salser, S.J. and Yamamoto, K.R. (1988). A movable and regulable inactivation function within the steroid binding domain of the glucocorticoid receptor. *Cell* **54** 1073-1080.

Robertson, R.S., Grossmann, S., Johannsen, E., Miller, C., Lin, J., Tomkinson, B. and Kieff, E. (1995). Epstein-Barr virus nuclear protein 3C modulates transcription through interaction with the sequence-specific DNA-binding protein Jκ. *J. Virol.* **69,** 3108-3116.

Rabson, M., Gradoville, L., Heston, L. and Miller, G. (1982). Non-immortalizing P3J-HR-1 Epstein-Barr virus: a deletion mutant of its transforming parent, Jijoye. *J. Virol.* **44,** 834-844.

Robertson. K.D., Barletta, J., Samid, D. and Ambinder, R.F. (1994). Pharmacologic activation of expression of immunodominant viral antigens: a new strategy for the treatment of Epstein-Barr-virus-associated malignancies. *Current Topics in Microbiol. and Immunol.* **194**, 145-154.

Rochford, R., Hobbs, M.V., Garnier, J.L., Cooper, N.L. and Cannon, M.J. (1993). Plasmocytoid differentiation of Epstein-Barr virus-transformed B cells in vivo is associated with reduced *Proc. Natl. Acad. Sci. U.S.A.* **90,** 352-356.

Rochford, R. and Mosier, D.E. (1995). Differential Epstein-Barr virus expression in B-cell subsets recovered from laymphomas in SCID mice after transplantation of human peripheral blood lymphocytes. *J. Virol.* **69,** 150-155.

Rowe, D.T., Rowe, M., Evan, G.I., Wallace, L.E., Farrell, P.J. and Rickinson, A.B. (1986). Restricted expression of EBV latent genes and T-lymphocyte-detected membrane antigen in Burkitt's lymphoma cells. *EMBO J.* **5,** 2599-2607

Spencer, C.A. and Groudine, M. (1991). Control of c-*myc* regulation in normal and neoplastic cells. *Adv. Cancer Res.* **56,** 1-48.

Sung, N.S., Kenney, S., Gutsch, D. and Pagano. J.S. (1991). EBNA-2 transactivates a lymphoid-specific enhancer in the BamHIC promoter of Epstein-Barr virus. *J. Virol.* **65,** 2164-2169.

Tomkinson, B., Robertson, E. and Kieff, E. (1993). Epstein-Barr virus nuclear proteins EBNA-3A and EBNA-3C are essential for B-lymphocyte growth transformation. *J. Virol.* **67,** 2014-2025.

Waltzer, L, Logeat, F., Brou, C., Israel, A., Sergant, A. and Manet, E. (1994). The human Jκ recombination signal sequence binding protein (RBP-Jκ) targets the Epstein-Barr virus EBNA2 protein to its DNA responsive elements. *EMBO J.* **13,** 5633-5638.

Wang, F., Gregory, C.D., Rowe, M., Rickinson, A.B., Wang, D., Birkenbach, M., Kikutani, H., Kishimoto, T. and Kieff, E. (1987). Epstein-Barr virus nuclear antigen 2 specifically induces expression of the B-cell activation antigen CD23. *Proc. Natl. Acad. Sci. U.S.A.* **84,** 3452-3456.

Wendel-Hansen, V., Rosén, A. and Klein, G. (1987). EBV-transformed lymphoblastoid cell lines down-regulate EBNA in parallel with secretory differentiation. *Int. J. Cancer* **39,** 404-408.

Woisetschlaeger, M., Jin, X.W., Yandava, C.N., Furmanski, L.A., Strominger, J.L. and Speck, S.H. (1991). Role of the Epstein-Barr virus nuclear antigen 2 in viral promoter switching during initial stages of infection. *Proc.* Natl. *Acad.* Sci. *U.S.A.* **88,** 3942-3946.

Yates, J., Warren, N., Reisman, D. and Sugden, B. (1984). A cis-acting element from the Epstein-Barr viral genome that permits stable replication of recombinant plasmids in latently infected cells. *Proc. Natl. Acad. Sci. U.S.A.* **81,** 3806-3810.

Young, L.S., Dawson, C.W., Clark, D., Rupani, H., Busson, T., Tursz, T., Johnson, A. and Rickinson, A.B. (1988). Epstein-Barr virus gene expression in nasophryngeal carcinoma. *J. Gen. Virol.* **69,** 1051-1065.

Zimber, U., Adldinger, H., Lenoir, G.M., Vuillaume, M., v. Knebel-Doeberitz, M., Laux, G., Desgranges, C., Wittmann, P., Freese, U.K., Schneider, U. and Bornkamm, G.W. (1986). Geographical prevalence of two types of Epstein-Barr virus. *Virology* **154,** 56-66.

Zimber-Strobl, U., Suentzenich, K.O., Laux, G., Eick, D., Cordier, M., Calender, A., Billaud, M., Lenoir, G.M. and Bornkamm, G.W. (1991). Epstein-Barr virus nuclear antigen 2 activates transcription of the terminal protein gene. *J. Virol.* **65,** 415-423.

Zimber-Strobl, U., Kremmer, E., Grässer, F., Marschall, G., Laux, G. and Bornkamm, G.W. (1993). The Epstein-Barr virus nuclear antigen 2 interacts with an EBNA2 responsive cis-element of the terminal protein 1 gene promoter. *EMBO J.* **12,** 167-175.

Zimber-Strobl, U., Strobl, L.J., Meitinger, C., Hinrichs, R., Sakai, T., Furukawa, T., Honjo, T. and Bornkamm, G.W. (1994). Epstein-Barr virus nuclear antigen 2 exerts its transactivating function through interaction with recombination signal binding protein RBP-Jκ, the homologue of drosophila suppressor of hairless. *EMBO J.* **13,** 4973-4982.

## Patentansprüche

1. Verfahren zur Herstellung von menschlichen monoklonalen Antikörpern mit folgenden Verfahrensschritten:
a) Immortalisierung menschlicher, Antikörper produzierender B-Zellen mit einem Epstein-Barr-Virus oder einem Derivat hiervon,
b) Screening der immortalisierten B-Zellen auf Spezifität für das gewünschte Antigen und Isolation von Zellklonen, die entsprechende Antikörper produzieren,
c) Gewinnung der gewünschten Antikörper aus den Kulturüberständen,
**dadurch gekennzeichnet, daß**
d) ein Epstein-Barr-Virus oder ein Derivat hiervon mit, in funktioneller Verbindung, einem konditionalen Epstein-Barr-Virus-Nuclear-Antigen 2-(EBNA2-)Gen oder einem Derivat hiervon verwendet wird, welches zur Immortalisierung zumindest teilweise angeschaltet wird, und
e) das EBNA2-Gen oder sein Derivat, in funktioneller Verbindung, vor Gewinnung der Antikörper zumindest teilweise abgeschaltet wird.

2. Verfahren nach Anspruch 1,
**dadurch gekennzeichnet, daß**
das EBNA2-Gen mit einer Hormonbindungsdomäne, in funktioneller Verbindung, fusioniert wird.

3. Verfahren nach Anspruch 1 oder 2,
**dadurch gekennzeichnet, daß**
als Hormonbindungsdomäne ein Östrogen- oder Androgenrezeptorgen verwendet wird.

4. Verfahren nach einem oder mehreren der vorhergehenden Ansprüche,
**dadurch gekennzeichnet, daß**
das EBNA2-Gen durch Zugabe des für die Hormonbindungsdomäne spezifischen Hormons zumindest teilweise angeschaltet und durch Entfernung des Hormons zumindest teilweise abgeschaltet wird.

5. Verfahren nach einem oder mehreren der vorhergehenden Ansprüche,
**dadurch gekennzeichnet, daß**
das EBNA2-Gen mit einer konditionalen Sequenz, bevorzugt einem konditionalen Promotor, fusioniert wird.

6. Verfahren nach einem oder mehreren der vorhergehenden Ansprüche,
**dadurch gekennzeichnet, daß**
primäre menschliche B-Zellen von Menschen, die Antikörper gegen ein gewünschtes Epitop (Antigen) tragen, verwendet werden.

7. Verfahren nach einem oder mehreren der vorhergehenden Ansprüche,
**dadurch gekennzeichnet, daß**
das EBNA2-Protein als Fusionsprotein mit der Hormonbindungsdomäne des Östrogenrezeptors exprimiert wird oder die Expression des EBNA2-Gens durch einen konditionalen Promotor reguliert wird.

8. Verfahren nach einem oder mehreren der vorhergehenden Ansprüche,
**dadurch gekennzeichnet, daß**
als Epstein-Barr-Virus-Derivat ein die zur Immortalisierung notwendigen Epstein-Barr-Virus-Sequenzen tragender Vektor verwendet wird.

9. Verfahren nach Anspruch 8,
**dadurch gekennzeichnet, daß**
als Vektor ein Mini-EBV-Plasmid verwendet wird.

10. Verfahren nach einem oder mehreren der vorhergehenden Ansprüche,
**dadurch gekennzeichnet, daß**
der Antikörper aus der Gruppe von IgM, IgG oder IgA ausgewählt wird.

11. Verfahren nach einem oder mehreren der vorhergehenden Ansprüche,
**dadurch gekennzeichnet, daß**
die Immortalisierung durch Infektion oder Transfektion der Antikörper produzierenden B-Zelle mit einem Epstein-Barr-Virus oder einem Derivat hiervon durchgeführt wird.

## Claims

1. Process for the preparation of human monoclonal antibodies comprising the following steps of:
a) immortalization of human antibody-producing B cells with an Epstein-Barr virus or a derivative thereof;
b) screening said immortalized B cells for specifity for the desired antigen and isolation of cellular clones producing corresponding antibodies;
c) recovery of the desired antibodies from the cultural supernatants;
**characterized in that**
d) said Epstein-Barr virus or a derivative thereof is used together with, in functional connection, a conditional Epstein-Barr virus nuclear antigen 2 (EBNA2) gene or a derivative thereof, at least partly switched on to achieve immortalization; and
e) the EBNA2 gene or its derivative, in functional connection, is at least partly switched off prior to recovery of said antibodies.

2. Process according to claim 1,
**characterized in that**
said EBNA2 gene is fused, in functional connection with a hormone binding domain.

3. Process according to claim 1 or 2,
**characterized in that**
an estrogen or androgen receptor gene is used as said hormone binding domain.

4. Process according to one or more of the preceding claims,
**characterized in that**
said EBNA2 gene is at least partly switched on by addition of the hormone specific for said hormone binding domain and is at least partly switched off by removal of said hormone.

5. Process according to one or more of the preceding claims,
**characterized in that**
said EBNA2 gene is fused with a conditional sequence and preferably a conditional promoter.

6. Process according to one or more of the preceding claims,
**characterized in that**
primary human B cells are used which are derived from humans bearing antibodies directed against a desired epitope (antigen).

7. Process according to one or more of the preceding claims,
**characterized in that**
said EBNA2 protein is expressed as fusion protein with the hormone binding domain of the estrogen receptor or the expression of said EBNA2 gene is controlled by a conditional promoter.

8. Process according to one or more of the preceding claims,
**characterized in that**
said Epstein-Barr virus derivative used is a vector bearing Epstein-Barr virus sequences necessary for immortalization.

9. Process according to claim 8,
**characterized in that**
a mini-EBV-plasmid is used as said vector.

10. Process according to one or more of the preceding claims,
**characterized in that**
said antibody is selected from the group consisting of IgM, IgG or IgA.

11. Process according to one or more of the preceding claims,
**characterized in that**
said immortalization is performed by infection or transfection of said antibody producing B cell with an Epstein-Barr virus or a derivative thereof.

## Revendications

1. Procédé de fabrication d'anticorps monoclonaux humains comportant les étapes suivantes :
a) immortalisation de cellules B humaines productrices d'anticorps avec un virus d'Epstein-Barr ou un dérivé de celui-ci,
b) dépistage des cellules B immortalisées présentant une spécificité vis-à-vis de l'antigène désiré et isolation de clones cellulaires, qui produisent les anticorps correspondants,
c) prélèvement des anticorps désirés à partir des cultures,
**caractérisé en ce que**
d) un virus d'Epstein-Barr ou un dérivé de celui-ci est utilisé en liaison fonctionnelle avec un gène conditionnel nucléaire antigène 2 d'Epstein-Barr (EBNA2) ou un dérivé de celui-ci, qui est au moins en partie activé pour l'immortalisation, et
e) le gène de la EBNA2 ou son dérivé, en liaison fonctionnelle, est au moins en partie désactivé avant le prélèvement des anticorps.

2. Procédé selon la revendication 1, **caractérisé en ce que** le gène de la EBNA2 est fusionné, en liaison fonctionnelle, avec un domaine de fixation hormonale.

3. Procédé selon la revendication 1 ou 2, **caractérisée en ce qu'**on utilise un gène du récepteur d'oestrogène ou d'androgène comme domaine de fixation hormonale.

4. Procédé selon l'une quelconque des revendications précédentes, **caractérisé en ce que** le gène de la EBNA2 est au moins en partie activé par l'ajout de l'hormone spécifique du domaine de fixation hormonale, et au moins en partie désactivé par la suppression de l'hormone.

5. Procédé selon l'une quelconque des revendications précédentes, **caractérisé en ce que** le gène de la EBNA2 est fusionné avec une séquence conditionnelle, de préférence avec un promoteur conditionnel.

6. Procédé selon l'une quelconque des revendications précédentes, **caractérisé en ce qu'**on utilise des cellules B primaires humaines, qui portent les anticorps contre un épitope (antigène) désiré.

7. Procédé selon l'une quelconque des revendications précédentes, **caractérisé en ce que** la protéine EBNA2 est exprimée comme protéine de fusion avec le domaine de fixation hormonale du récepteur de l'oestrogène ou l'expression du gène de la EBNA2 est régulée par un promoteur conditionnel.

8. Procédé selon l'une quelconque des revendications précédentes, **caractérisé en ce qu'**un vecteur porteur des séquences du virus d'Epstein-Barr nécessaires à l'immortalisation est utilisé comme dérivé du virus d'Epstein-Barr.

9. Procédé selon la revendication 8, **caractérisé en ce qu'**un mini-plasmide d'EBV est utilisé comme vecteur.

10. Procédé selon l'une quelconque des revendications précédentes, **caractérisé en ce que** les anticorps sont choisis parmi le groupe constitué de IgM, IgG ou IgA.

11. Procédé selon l'une quelconque des revendications précédentes, **caractérisé en ce que** l'immortalisation est réalisée par infection ou transfection des cellules B productrices d'anticorps avec un virus d'Epstein-Barr ou un dérivé de celui-ci.
